(19) Europäisches Patentamt
European Patent Office
Office européen des brevets

(11) **EP 3 795 137 B1**

(12) **EUROPEAN PATENT SPECIFICATION**

(45) Date of publication and mention
of the grant of the patent:
**10.08.2022 Bulletin 2022/32**

(21) Application number: **19197645.5**

(22) Date of filing: **17.09.2019**

(51) International Patent Classification (IPC):
*A61K 8/34* (2006.01)　　　*A61K 8/81* (2006.01)
*A61K 8/88* (2006.01)　　　*A61Q 17/04* (2006.01)

(52) Cooperative Patent Classification (CPC):
**A61K 8/34; A61K 8/8158; A61K 8/88; A61Q 17/04;**
A61K 2800/31; A61K 2800/805

(54) **ELECTROSTATICALLY SPRAYABLE COSMETIC FORMULATION**

ELEKTROSTATISCH SPRÜHBARE KOSMETISCHE FORMULIERUNG

FORMULATION COSMÉTIQUE PULVÉRISABLE ÉLECTROSTATIQUEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**24.03.2021 Bulletin 2021/12**

(73) Proprietor: **IONIQ Skincare GmbH & Co. KG
88677 Markdorf (DE)**

(72) Inventors:
- **GÖHRING, Alfred
  88682 Salem (DE)**
- **BARTHELMES, Jan
  88682 Salem (DE)**
- **JELTSCH, Thomas
  88048 Friedrichshafen (DE)**
- **LANGEN, Valentin
  88662 Überlingen (DE)**
- **LUCK, Leon
  88677 Markdorf (DE)**
- **STOHL, Holger
  88677 Markdorf (DE)**
- **VERDICCHI, Charlotte
  22927 Großhansdorf (DE)**

(74) Representative: **Lederer & Keller Patentanwälte
Partnerschaft mbB
Unsöldstraße 2
80538 München (DE)**

(56) References cited:
　　**WO-A1-2018/194086　　DE-U1-202010 006 005**

- **"SYLVASOL Polymers - Ethanol Sunscreen
  Sprays", IP.COM JOURNAL, IP.COM INC., WEST
  HENRIETTA, NY, US, 21 February 2012
  (2012-02-21), XP013149383, ISSN: 1533-0001**
- **DATABASE GNPD [Online] MINTEL; 2 April 2019
  (2019-04-02), anonymous: "Beautifying Oil
  SPF30", XP055673562, retrieved from
  www.gnpd.com Database accession no. 6448199**

**Description**

The present invention relates to an electrostatically sprayable cosmetic formulation, which can be easily applied on a subject and provides in particular an improved protection against harmful UV-A and UV-B radiation and additionally a good water resistance.

It is well known that ultraviolet radiation of the sun having a wave length of about 280 to about 400 nm, i.e. UV-B (280 nm to 320 nm) and UV-A radiation (320 nm to 400 nm), is responsible for skin damages such as sunburn or cellular mutation, but also causes loss of skin elasticity, suppress immune functions, promote premature signs of aging and other undesirable health effects. On the other hand, for most of the people is it desirable to tan the skin for purpose of appearance. The tanning of the skin is caused by ultraviolet radiation of longer wave lengths of up to about 420 nm. In order to prevent or reduce the negative impacts of the sun radiation various sun screen products are on the market. In addition to this main function of the sun screen it is also desirable that the sun screen shows a good resistance against sweat, fresh or salt water in case the subject is exposed to water.

Nowadays, sun screens are commercial available having a high protection against UV-A and UV-B sun radiation and which show a good water resistance.

DE 20 2010 006 005 U1 refers to a clear sun screen formulation having a high light protection.

However, even though from a theoretical point of view the sun screen formulations known in the prior art show sufficient protection against sun radiation, in practice the protection against harmful sun radiations leaves much to be desired. One of the reasons for this is that the sun screen formulations are not applied on the surface of the skin in a correct manner.

Most of the sun screen formulations are lotions which can be applied on the surface of the skin by a limiting number of methods. The simple method is to apply the lotion by hand. This method includes many disadvantages. In particular, the method does not ensure that a sufficient amount of the sunscreen lotion is applied on the surface of the skin and in particular this application method does not provide a sufficient coverage of the skin. Moreover, in some cases unnecessary high amounts of the sun screen formulation are applied on skin surface which results into uncomfortable feeling.

Another application method, which should ensure that a sufficient amount of the sun screen formulation is applied on the surface of the skin, but in an adequate manner, is the use of pump sprays or pre-pressurized aerosol containers so as to have the formulation atomized and sprayed with the aid of propellant gas. In the publication "SYLVASOL Polymers - Ethanol Sunscreen sprays" IP.COM Journal, IP.COM Inc., West Henrietta, NY, US, 2012-02-21, and in database GNPD (online) Mintel, 2019-04-02, anonymous, "Beautifying Oil SPF30", sun screen formulations are described, which are sprayable. However, conventional aerosol sprays frequently employ volatile compounds as propellants, which are environmentally unfriendly, possible hazardous to health and indeed are being legislated against in many countries. Moreover, it is hardly possible to generate a constant and homogenous flow of the sun screen formulation. Therefore, also with this application method a sufficient coverage of the skin surface, i.e. a nearly 100% coverage, is not possible. In the last few years in the field of cosmetic products a further application method has become of interest. In this application method the cosmetic product is applied on the subject by electrostatic spraying. Such a method is for example described in WO 94/11119 or WO 2001/012139.

WO 2018/194086 refers to a coating film, which provides an ultraviolet protection effect and can be applied on the skin by electrostatic spraying.

This application method can be carried out without the aid of environmentally unfriendly propellants and provides a constant and uniform flow of the product. Hence, it is possible to apply a sufficient amount of the cosmetic formulation, e.g. sun screen formulation, on for example the surface of the skin and thus a nearly full coverage of the skin may be possible.

In the prior art different electrostatic spraying devices are known, for example in WO 94/11119, US 2010/0116897 or DE 10 2017 108 610.2 such devices are described. In an electrostatic spaying device a voltage generator creates a high voltage which electrically charges the compounds of the cosmetic product. This results into a spray of the cosmetic product.

However, the most problem of this application method is that the known cosmetic formulations such as sun screen formulations are not electrostatically sprayable, since they do not have appropriate electrical characteristics, e.g. resistivity, permittivity etc., and/or have other properties such like a high surface tension, viscosity etc. which do not permit electrostatic spraying. For example, like many other cosmetic lotions and creams, sun screen formulations include in addition to UV-A and UV-B filters emollients.

Emollients, also referred to as cosmetic oils, are compounds that soften and smooth the scales of the skin, which help to reduce rough and flaky skin. They are frequently occlusive agents, i.e. substances that provide a layer of protection that help prevent moisture (water) loss from the skin. Examples for emollients are silicones, such as dimethicone or cyclomethicone, vegetable oils, butters such as coca butter or shea butter, alcohols such as stearyl alcohol or cetyl alcohol, and petrolatum derivatives such a petroleum jelly or mineral oil. In sun screen formulations emollients are additionally used as solubilizer for crystalline UV filters. Due to their electrical characteristic, i.e. resistivity and permittivity, most of the emollients show a worse electrostatic sprayability. The same applies to the UV filters which are usually used in sun screen formulations.

Moreover, even though some of the cosmetic formulations have appropriate electrical characteristics, it is still not possible to electrostatically spraying these formulations such that all parts of the skin are covered by the cosmetic formulation. This result into a so-called streaking effect on the skin, i.e. the applied cosmetic formulation creates a "zebra crossing" pattern on the skin.

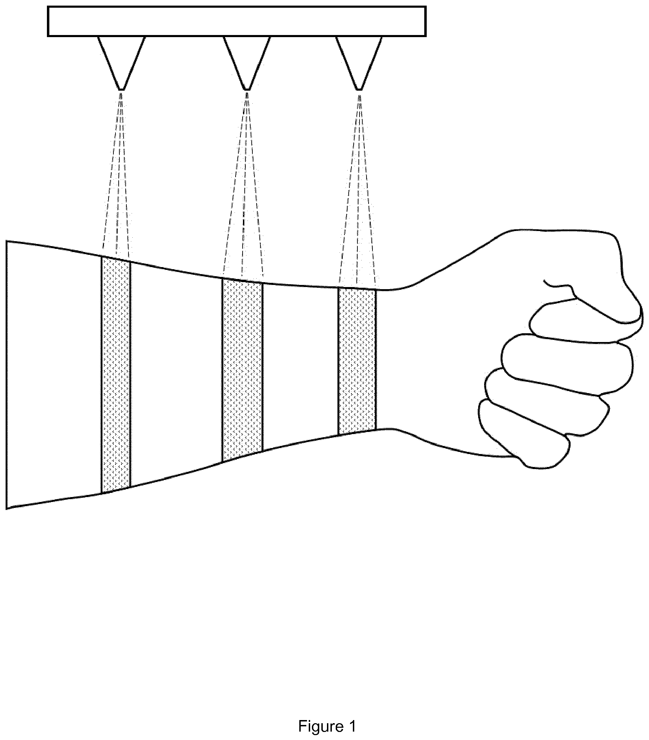

Figure 1 schematically shows the undesired "zebra crossing" pattern on the skin, which occurs if the cosmetic formulation is not uniformly sprayable and thus, no homogenous distribution of the formulation on the skin is possible.

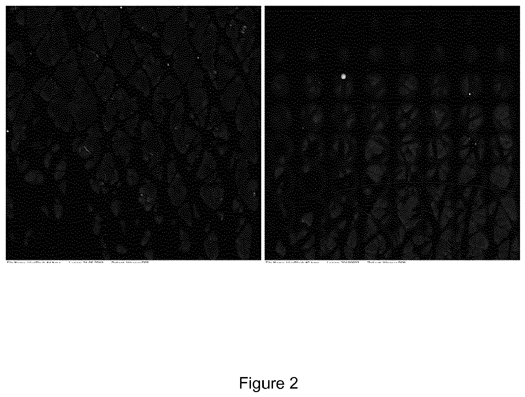

Figure 2 shows the untreated skin surface of forearms of two subjects (reference measurement).

Figures 3-5 show the skin surfaces of forearms of subjects treated with the Formulations of Comparative Examples 1, 2 and 3 according to spray test 1 as described below.

Thus, object of the application was to provide an electrostatically sprayable cosmetic formulation that has a sun protection efficiency comparable to sun screen formulations known in the prior art and additionally provides a nearly 100 % coverage of the surface of the skin, i.e. the distribution of the formulation on the skin is homogenous and thus no streaking effect occurs.

It has been surprisingly found that in case the cosmetic formulation comprises ethanol and at 20 °C liquid polar emollients (cosmetic oils) in combination with a film forming agent mixture, which comprises at least one polyalkyleneoxy terminated polyamide and at least one acrylate, the cosmetic formulation shows an improved electrostatic sprayability, i.e. in particular, no streaking effect occurs and thus a nearly full coverage of the skin can be achieved.

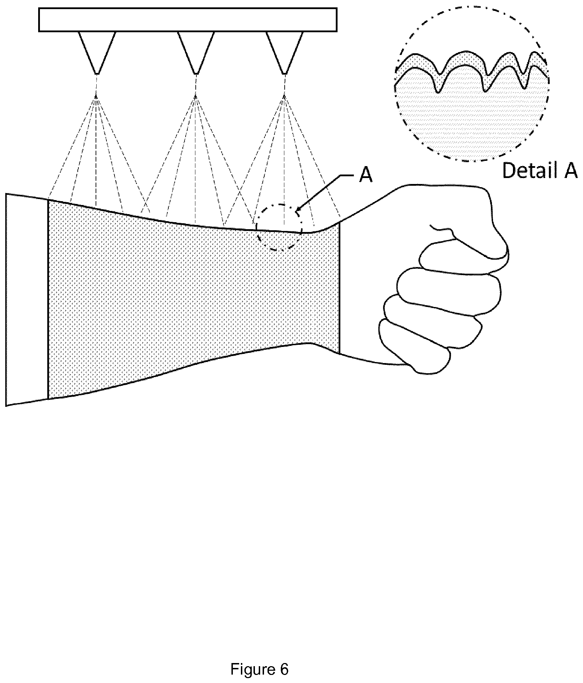

Figure 6 schematically shows the full coverage of the skin by using a cosmetic formulation of the invention, i.e. no streaking effect occurs. The enlarged section "Detail A" of Figure 6 shows a part of the skin, which is covered with the cosmetic formulation according to the invention.

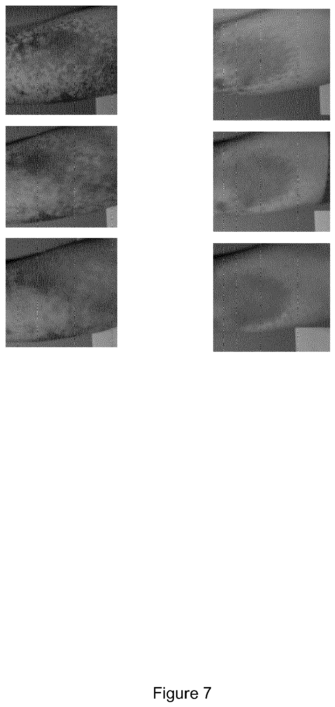

Figure 7 shows the skin surface of forearms of subjects treated with the Formulation of Comparative Example 4 (left side) and treated with the inventive Formulation SPF 30 (right side) according to the spray test 2 as described below.

According to the claimed invention, in order to ensure that the cosmetic formulation is electrostatic sprayable, ethanol has to be used in a sufficient amount in combination with liquid polar emollients, whereby the polarity of the emollients is given by their log $K_{ow}$ value.

Furthermore, it has been found out that the use of a film forming agent mixture in the cosmetic formulation, which comprises a polyalkyleneoxy terminated polyamide in combination with an acrylate, improves the electrostatic sprayability of the cosmetic formulation, although the amount of ethanol used in the formulation is in the middle range, and further results into an increase of the sun protection factor (SPF) of the cosmetic formulation.

Additionally, it has been found out that by using the film forming agent mixture according to the invention the use of a thickening agent in the cosmetic formulation is not necessary.

Therefore, the present invention provides an electrostatically sprayable cosmetic formulation, which comprises

a) 20 to 60 wt.-% ethanol;
b) 10 to 55 wt.-% emollients, which are liquid at 20 °C and have a log $K_{ow}$ of 2.0 to 10;
c) 10 to 55 wt.-% of UV-A and/or UV-B filters;
d) 0.5 to 15 wt.-% of a polymeric film forming agent mixture comprising at least one polyalkyleneoxy terminated polyamide and at least one acrylate; and
e) 0 to 4 wt.-% of cosmetic additives, based on the total weight of the formulation,

wherein the water content present in the formulation is less than 5 wt.-%, based on the total weight of the formulation, and the sum of the weight of the components is 100 %.

The cosmetic formulation as defined above shows the desired electrostatic spraying behavior, i.e. no striking effect occurs, an improved sun protection factor (SPF) and a good water resistance.

The abbreviation "wt.-%" or "w/w", as used herein, means "weight percentage" and refers to the weight amount of a compound in relation to the (total) weight of a formulation of compounds or of a substrate if nothing else is explicitly stated or obvious under the circumstances.

The term "comprising" includes "consisting essentially of' and also "consisting of".

In the present specification, the use of "a" in the singular also comprises the plural ("some"), and vice versa, unless the context clearly indicates the contrary.

The cosmetic formulation in the meaning of the invention is a formulation that is selected from the group consisting of sun screen, skin care, hairspray, cosmetic foundation, lipstick, hair dye, and self-tanning formulations. The cosmetic formulation can be used alone or in a mixture comprising other cosmetic formulations. Preferably, the cosmetic formulation is a sun screen formulation.

The electrostatically sprayable cosmetic formulation having the specific combination of components according to the invention preferably shows an specific electrical resistance of 15 to 100 GOhm·mm$^2$/m, more preferably of 20 to 85 GOhm·mm$^2$/m, it is even more preferred that the specific electrical resistance is not higher than 80, not higher than 60, not higher than 50 GOhm·mm$^2$/m, most preferably the electrostatic resistance is between 35 to 48 GOhm·mm$^2$/m, determined as described below (see measurement methods). This ensures that the formulation is good electrostatically sprayable and thus no undesired streaking effect on the skin occurs.

In addition, in order to provide a cosmetic formulation which shows a good electrostatic spraying behavior, the formulation preferably has a surface tension of 15 to 50 mN/m, more preferably of 20 to 45 mN/m, 22 to 40 mN/m, more preferably 25 to 35 mN/m determined by the stamp method as described below (see measurement methods).

Furthermore, the relative permittivity of the cosmetic formulation according to the invention may be at least 2.0, preferably at least 2.3, more preferably at least 2.5, most preferably at least 2.6. In particular, the relative permittivity of cosmetic formulation preferably is between 2.4 and 25.0, more preferably between 2.4 and 20.0, or between 2.4 and 10.0, most preferably between 2.4 and 5.0. The measurement method for determining the relative permittivity according to the invention is described below (see measurement methods).

Moreover, the cosmetic formulation of the invention may have a viscosity of from 0.65 mPa·s to 5000 mPa·s, preferably of from 1.0 to 1000 mPa·s, 5.0 to 500 mPa·s, more preferably of from 10.0 to 100 mPa*s, determined as described below (see measurement methods). In particular a good spraying behavior of the cosmetic formulation can be observed if the cosmetic formulation has a viscosity below 4.0 mPa·s. A cosmetic formulation having a viscosity of at least 8.0 mPa·s shows a sufficient spraying behavior.

For spaying the cosmetic formulation on the subject every electrostatic device known in the art can be used. However, it is preferred to use a device as described in DE 10 2017 108 610.2, which is further specified in DE 10 2017 108 612.9, DE 10 2017 108 613.7, DE 10 2107 108 614.5 and DE 10 2107 108 615.3.

As mentioned above, in order to ensure that the cosmetic formulation has the required electrostatic characteristics and thus shows a good electrostatic spraying behavior the formulation comprises ethanol in a sufficient amount in combination with liquid polar emollients.

It has been further found out that in addition to the sprayability of the emollients and thus of the total cosmetic formulation, also the sprayability of other components present in the cosmetic formulation can be positively influenced by the presence of ethanol in the formulation. For example, the highly viscous UV-B filter octocrylene shows a worse electrostatic sprayability in particular at low voltages. However, in case octocrylene is mixed with ethanol, the spraying quality can be improved (see results of Table 1 below). The spraying test was carried out such that the distance from a spraying nozzles to the outer left side of a metal tube was 13 cm. The pre-set voltage of three subsequent tests were 20, 30 and 40 kV, respectively. For the fluid pump a voltage of 3.7 V was used. After changing each test liquid, the tube system was rinsed with isopropanol and/or ethanol. The spraying behavior of the formulations was optically evaluated and the spraying pattern was assessed to the following scale: good; moderate, poor and nearly not sprayable.

| Formulation | Electrical conductivity [μm/cm] (at °C) | Surface tension [mN/m] (°C) (bubble pressure method; at 20s bubble lifetime) | Spraying results | | |
|---|---|---|---|---|---|
| | | | Pre-set 20 kV | Pre-set 30 kV | Pre-set 40 kV |
| Octocrylene (1 %)/ Ethanol (99 %) | 0.5 (25.6 °C) | n.d. | nearly no spraying at 16 kV | moderate spraying at 23 kV | moderate to good spraying at 29 kV |
| Octocrylene (10 %) / Ethanol (90 %) | 0.7 (22.6 °C) | 22.34 (26.8 °C) | nearly no spraying at 16 kV | poor to moderate spraying at 23 kV | moderate to good spraying at 30 kV |
| Octocrylene (100 %) | 0.0 (at 23.7 °C) | Too viscous n.d. | nearly no spraying at 16 kV | nearly no spraying at 16 kV | poor to moderate spraying at 34 kV |
| n.d. not determined Table 1 | | | | | |

As a general trend it can be seen that the spraying quality raises with the ethanol content. However, a high ethanol content in cosmetic formulations, in particular in sun screen formulations, is not desired since ethanol shows the tendency of drying out and irritating the skin.

According to the invention, the amount of ethanol present in the cosmetic formulation can be reduced, in case ethanol is used in combination with emollients and the film forming agent mixture as defined in the invention. In that case, an ethanol content of 20 to 60 wt.-%, based on the total weight of the formulation, is sufficient. In particular, it is preferred that ethanol content is at least 25 wt.-%, at least 35 wt.-%, at least 40 wt.-%, more preferably at least 45 wt.-%, but not higher than 58 wt.-%, not higher than 55 wt.-%, more preferably not higher than 52 wt.-%, based on the total weight of the formulation. In a preferred embodiment of the invention, the ethanol content is between 50 and 55 wt.-%, based on the total weight of the formulation. In another preferred embodiment, the ethanol content is between 42 and 46 wt.% or between 38 and 42 wt.-%, based on the total weight of the composition.

The emollients (cosmetic oil) of the invention are emollients which are usually used in cosmetic products such like sun screen formulations, i.e. substance that provide a layer of protection that helps prevent moisture (water) loss from the skin as described above. It is further required that the emollients are liquid at 20 °C and polar.

In the cosmetic field, one possibility to define the polarity of a compound is to determine the n-octanol/water partition coefficient ($K_{ow}$ or P). The partition-coefficient refers to the ratio of concentrations of the compounds in the mixture of these two immiscible phases at equilibrium. Hence, the partition coefficient measures how hydrophilic ("water-loving") or hydrophobic ("water-fearing") the tested compound is. In most of the cases the n-octanol/water partition coefficient is stated as decade logarithms log $K_{ow}$ or log P. The determination of the n-octanol/water partition coefficient is described for example in J. Sangster, "Octanol-Water Partition Coefficients: Fundamentals and Physical Chemistry", Vol. 2 of Wiley Series in solution chemistry, John Wiley & Sons, Chichester, 1997.

According to the invention, the log $K_{ow}$ of the emollients, which are suitable for the cosmetic formulation of the invention, is between 2.0 to 10.0, preferably between 2.5 and 9.5, 3.0 and 8.5, more preferably between 3.5 and 7.5.

An emollient known in the prior art which fulfills the above discussed requirements is suitable for the cosmetic formulation of the invention. Examples of suitable emollients are: phenoxyethyl caprylate, PPG-3 myristyl ether, dibutyl adipate, dicaprylyl carbonate, isoamyl cocoate, diethylhexyl carbonate, isopropyl myristate, isopropyl palmitate, PPG-14 butyl ether, decyl cocoate, PPG-15 stearyl ether, C12-C15 alkyl benzoate and combinations thereof.

It is preferred that the cosmetic formulation of the invention comprises at least two or more, at least three, at least 4, but not more than 10, preferably not more than 8, more preferably not more than 5 different emollients. In particular, it is preferred that the cosmetic formulation comprises phenoxyethyl caprylate, PPG-3 myristyl ether, dibutyl adipate, dicaprylyl carbonate and combinations thereof as emollients. In particular, it is preferred that the cosmetic formulation of the invention comprises a mixture consisting of these four emollients.

The different emollients are used in different or equal amounts in the formulation so that the total amount of emollients is between 10 to 55 wt.-%, based on the total weight of the formulation. Preferably, the total amount of emollients present in the formulation is from 15 to 50 wt.-%, 20 to 45 wt.-%, 22 to 40 wt.-%, 23 to 30 wt.-%, based on the total weight of the formulation.

The cosmetic formulation of the invention shows a sufficient sun screen protection. This property is indicated by the so-called sun protection factor (SPF). The SPF is a measure of the fraction of sunburn producing UV radiation that reach the skin. For example, "SPF 15" means that 1/15th of the burning radiation will reach the skin, assuming sunscreen is applied evenly at a thick dosage of 2 milligrams per square centimeter. The determination of the SPF of a sun screen formulation is described in detail in ISO 24444:2010 (E).

The cosmetic formulation of the invention has a SPF of at least 15, preferably of at least 20, of at least 25, of least 30, more preferably of at least 50 or higher, measured according to ISO 24444:2010 (E). Sun screen formulations which have a SPF of 60 and higher are indicated as SPF 50+ sun screen formulations. It is most preferred that the cosmetic formulation of the invention has a SPF of at least 30.

The ability to protect the subject from harmful radiation of the sun depends inter alia on the amount of UV-A and UV-B filters present in the cosmetic formulations. In general, a mixture of UV-A and UV-B filters are used. In the present invention, the cosmetic formulation comprises UV-A and/or UV-B filters in an amount of 10 to 55 wt.-%, preferably of 12 to 50 wt.-%, 15 to 40 wt.-%, 20 to 35 wt.-%, more preferably between 16 and 32 wt.-% or between 18 and 31 wt.-%, based on the total weight of the formulation.

Moreover, with respect to a good electrostatic sprayability of the formulation, it is preferred that the UV-A and/or UV-B filters have a log $K_{ow}$ of between 2.0 to 7.0, preferably between 2.5 and 6.5, more preferably between 3.0 and 6.0.

As mentioned above, in general a mixture of UV-A and UV-B filters are used in sun screen formulations. This is also the case for the cosmetic formulation of the invention, whereby the amount of UV-A and UV-B filters can be equal or different. Preferably, the concentrations of the UV-A and B filters used in the cosmetic formulation of the invention are in accordance with the requirements according to Regulation (EC) No. 1223/2009 on cosmetic products, Annex VI.

In particular it is preferred that the cosmetic formulation comprises at least 4, at least 5 but not more than 10, preferably not more than 8 different UV-filters which can be UV-A and/or UV-B filters.

Any UV-filter known in the prior art and which does not negatively influence the electrostatic sprayability of the formulation can be used in the invention. Suitable UV filters are disclosed in Regulation (EC) No. 1223/2009 on cosmetic products, Annex VI. Some of these examples are: phenylene-1,4-bis-(2-benzimidazyl)-3,3'-5,5'-tetrasulfonacid salts; phenylbenzimidazole sulfonic acid; 2-phenylbenzimidazole-5-sulfonic acid salts; 1,4-di(2-oxo-10-sulfo-3-bornylidenemethyl) benzene and its salts; 4-(2-oxo-3-bornylidenemethyl)-benzenesulfonic acid; 2-methyl-5-(2-oxo-3-bornylidenemethyl) sulfonic acid salts; 2,2'-methylene-bis- (6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]-disiloxanyl] propyl] phenol; 3-(4-methylbenzylidene)-camphor; ethylhexyl salicylate; terephthalidendicamphersulfonic acid; 4-(dimethylamino) benzoic acid (2-ethylhexyl) ester; 4-(dimethylamino) benzoic acid amyl ester; 4-methoxybenzalmalon-di(2-ethylhexyl) ester; 4-methoxycinnamate (2-ethylhexyl) ester, 4-methoxycinnamate; 2-hydroxy-4-methoxybenzophenone; 2-hydroxy-4-methoxy-4'-methylbenzophenone; 2,2'-dihydroxy-4-methoxybenzophenone; hexyl-2-(4'-diethylamino-2'-hydroxybenzoyl) benzoate 4-(tert-butyl)-4'-methoxydibenzoylmethane; homomethylsalicylate (INCI: Homosalate); 2-ethylhexyl-2-hydroxybenzoate; 2-ethylhexyl-2-cyano-3,3-diphenylacrylate (INCI: Octocrylene); dimethicodiethylbenzalmalonate; 3-(4-(2,2-ethoxycarbonyl-vinyl)-phenoxy)-propenyl)-methoxy-siloxane/dimethylsiloxane copolymer; 2,4-bis-{[4- (2-ethyl-hexyloxy)-2-hydroxy]-phenyl}-6-(4-methoxyphenyl)-1,3,5-triazine (INCI: bis-ethylhexyloxyphenol methoxyphenyl triazine); dioctylbutylamido triazone (INCI: diethylhexyl-butamido triazone); 2,4-bis-[5-1-(dimethylpropyl)benzoxazol-2-yl-(4-phenyl)-imino]-6-(2-ethylhexyl)-imino-1,3,5-triazine having the (CAS No 288254-16-0); ethylhexyl triazine; 4,4',4"-(1,3,5-triazine-2,4,6-triyltriimino)-tris-benzoic acid-tris-2-ethylhexyl ester (also: 2,4,6-tris[anilino-(p-carbo-2'-ethyl-1'-hexyloxy)]-1,3,5-triazine (INCI: ethylhexyl triazone); 2,4,6-tribiphenyl-4-yl-1,3,5-triazine; butyl-methoxydibenzoylmethane; isoamyl-p-methoxycinnamate and diethylamino hydroxybenzoyl hexyl benzoate.

Preferably, the UV-filters are selected from the group consisting of homosalate, butyl methoxydibenzoylmethane, octocrylene, diethylhexyl butamido triazone, ethylhexyl salicylate, bis-ethylhexyloxyphenol methoxyphenyl triazine, diethylamino hydroxybenzoyl hexyl benzoate and combinations thereof.

In particular, it is preferred that the cosmetic formulation comprises a mixture of UV-A and UV-B filters which consists of butyl methoxydibenzoylmethane, octocrylene, diethylhexyl butamido triazone, ethylhexyl salicylate, optionally bis-ethylhexyloxyphenol methoxyphenyl triazine, optionally diethylamino hydroxybenzoyl hexyl benzoate, and optionally homosalate.

In addition to the specific combinations of ethanol, emollients and UV-A/UV-B filters as described above, according to the invention, in order to improve the sprayability of the cosmetic formulation the cosmetic formulation has to comprise a film forming agent mixture that comprises at least one polyalkyleneoxy terminated polyamide and at least one acrylate. Furthermore, the use of the film forming agent mixture according to the invention allows the SPF to be increased without the need of increasing the amount of UV filters present in the formulation and thus to maintain the good sprayability of the formulation.

Every known polyalkyleneoxy terminated polyamide, as for example described in US 2014/0212363, can be used in the present invention. However, it is preferred that the polyalkyleneoxy terminated polyamide is a condensation product of dilinoleic acid, ethylenediamine and polypropylene glycol diamine end-capped with polyethylene glycol-poylpropylene glycol (PEG-PPG)-32/10 aminopropyl methyl ether. It is more preferred that the polyalkyleneoxy terminated polyamide is selected from the group consisting of a polyamide-3, a polyamide-4, and mixtures thereof, such as OleoCraft MP-30, OleoCraft MP-32, OleoCraft HP-31, OleoCraft HP-33, SYLVASOL 80, SYLVACLEAR AF1900V, SYLVACLEAR PE1800V, SYLVACELEAR PA1200V and SYLVACLEAR WF1500V available for instance from Arizona Chemical or Croda. Preferably, the polyalkyleneoxy terminated polyamide is polyamide-3.

Furthermore, although, according to the invention, the acrylate can be any one known in the art, such as for example acrylate/dimethicone copolymer, styrene acrylate copolymer or vinyl acetate (VA)/butyl maleate/isobornyl acrylate copolymer, it is preferred that the acrylate is an acrylate/amide copolymer. More preferably, the acrylate/amide copolymer is an acrylate/amide copolymer with alkyl groups having 6 to 16 carbon atoms. Most preferably, the acrylate/amide copolymer is an acrylates/octylacrylamide copolymer. This polymer comprises carboxyl groups attached to the polymer main chain and octyl side chains and is commercially available, for example under the trademark DERMACRYL 2.0 or DERMARYL 79 from AkzoNobel, DE.

According to the invention, in addition to the polyalkyleneoxy terminated polyamide and the acrylate, the film forming agent mixture may contain as film forming agents monomer, copolymers, cross polymers and terpolymer of organ-silicone hybrid, biopolymer, abietic acid derivatives, polyolefins, silicone resins as known in the prior art, in particular vinyl acetates, maleates, alkyl esters, long chain and short chain carboxylic acids, polyamides, ethanol (and) crotonic acid/vinyl $C_{8-12}$ esters/VA/bis-vinyldimethicone crosspolymer, isododence (and) acrylate/dimethicone copolymer, acrylates/dimethicone copolymer (and) cyclopentasiloxane, methyl dihydroabietate, hydrogenated polycyclopentadiene isoalkyl (and) isododecane, vinylpyrollidone (VP)/hexadecane copolymer hydrogenated dimer dilinoleyl/diemethylcarbonate copolymer, trimethylpentanediol/adipic acid/glycerin crosspolymer, or combinations thereof.

As mentioned above, the cosmetic formulation of the invention comprises the polymeric film forming agent mixture in an amount of 0.5 to 15 wt.-%, based on the total weight of the formulation; preferably, in an amount of between 0.7 and 13 wt.-%, between 1 and 10 wt.-%, between 2 and 8 wt.-%, between 3 and 7 wt.-% and more preferably between 4 and 6 wt.-%, based on the total weight of the formulation.

Furthermore, according to the invention, it is preferred that the amount of the polyalkyleneoxy terminated polyamide present in the film forming agent mixture is higher than the amount of the acrylate, preferably the amount of the polyalkyleneoxy terminated polyamide is twice, triple or fourfold higher than the amount of acrylate used in the film forming agent mixture.

In particular, the cosmetic formulation comprises the polyalkyleneoxy terminated polyamide in an amount of 0.5 to 10 wt.-%, preferably in an amount of 1 to 8 wt.-%, 2 to 6 wt.-%, more preferably in an amount of 3 to 5 wt.-%, based on the total weight of the formulation.

The acrylate is preferably used in the cosmetic formulation in an amount of 0.2 to 3 wt.-%, preferably in an amount of 0.5 to 2 wt.-%, more preferably in an amount of 1 to 1.5 wt.-%, based on the total weight of the formulation.

It is most preferred that the amount of the polyalkyleneoxy terminated polyamide present in the cosmetic formulation of the invention is between 2 and 4 wt.-% and the amount of the acrylate is between 0.5 and 2 wt.-%, based on the total weight of the formulation.

In a preferred embodiment of the invention, the film forming agent mixture comprises (consists of) 1 to 5 wt.-% of a polyalkyleneoxy terminated polyamide and 0.5 to 2.5 wt.-% of an acrylate, based on the total weight of the formulation. In another embodiment, the film forming agent mixture consists of 3 wt.-% of a polyalkyleneoxy terminated polyamide and 1 wt.-% of an acrylate.

The water resistance of the cosmetic formulation according to the invention is preferably above 60 %, more preferably above 70 %, even more preferably above 80%, most preferably above 90 %, as usually determined and described below (see measurement methods).

In common, cosmetic formulations comprise in addition to the above described active compounds also cosmetic additives such as cosmetically acceptable carriers, oils, sterols, amino acids, moisturizers, powders, colorants, pigments, dyes, pH adjusters, perfumes, essential oils, vitamins E, pro-vitamins, essential fatty acid, sphingolipids, self-tanning compounds, etc. and combinations thereof. These additives and combinations thereof may be also present in the cosmetic formulation of the invention.

Preferably, according to the invention, the additives are selected from the group consisting of perfumes, moister agent such as glycerin, vitamins and/or pro-vitamins such as tocopherol and/or tocopheryl acetate, and combinations thereof.

The cosmetic formulation of the invention comprises 0 to 4 wt.-% of cosmetic additives, preferably 0.1 to 3 wt.-%, more preferably 0.2 to 2 wt.-%, based on the total weight of the formulation.

Furthermore, the amount of water present in the cosmetic formulation of the invention is less than 5 wt.-%, preferably less than 3 wt.-%, less than 1 wt.-%, more preferably less than 0.1 wt.-%, based on the total weight of the formulation. It is preferred that the water content of the formulation of the invention is not higher than 2.5 wt.-%, more preferably not higher than 0.5 wt.-%. In particular, it is preferred that the cosmetic formulation of the invention is water free, i.e. the water content of the cosmetic formulation of the invention is zero. According to the invention, the term "water free" includes that each compound used in the cosmetic formulation contains no residues of water, for example it is preferred that denaturized ethanol, which does not contain water, or water free glycerin (Glycerin Ph. Eur. 99%) is used in the cosmetic formulation of the invention.

Furthermore, since the compounds used in the cosmetic formulation of the invention, in particular the active compounds such as UV-filters, are completely soluble in the formulation of the invention, the formulation of the invention has an improved stability, in particular with respect to storage, and thus there is no need to use thickening agents as described for example in WO 01/12139 in the cosmetic formulation of the invention. Thickening agents are for example silicones, waxes, clays, silicas, salts, natural and synthetic esters, fatty alcohols, and mixture thereof.

In a further embodiment of the invention the electrostatically sprayable cosmetic formulation comprises

a) 35 to 55 wt.-% ethanol;
b) 18 to 27 wt.-% a emollients mixture, wherein the emollients are liquid at 20 °C and have a log $K_{ow}$ of 2.0 to 10;
c) 16 to 40 wt.-% of a UV-A/UV-B filter mixture;
d) 2 to 5 wt.-% of a polymeric film forming agent mixture comprising at least one polyalkyleneoxy polyamide and at least one acrylate, wherein the amount of polyalkyleneoxy polyamide is at least twice higher than the amount of acrylate present in the film forming agent mixture; and
e) 1 to 4 wt.-% of a cosmetic additive mixture;

wherein the water content present in the formulation is less than 1 wt.-%, based on the total weight of the formulation, and the sum of the weight of the components is 100 %.

In a further preferred embodiment of the invention, the electrostatically sprayable cosmetic formulation comprises

a) 35 to 55 wt.-% ethanol;
b) 18 to 27 wt.-% a emollients mixture which consists of phenoxyethyl caprylate, PPG-3 myristyl ether, dibutyl adipate and dicaprylyl carbonate;

c) 16 to 40 wt.-% of a UV-A/UV-B filter mixture which consists of butyl methoxydibenzoylmethane, octocrylene, diethylhexyl butamido triazone, ethylhexyl salicylate, optionally bis-ethylhexyloxyphenol methoxyphenyl triazine, optionally diethylamino hydroxybenzoyl hexyl benzoate and optionally homosalate;

d) 2 to 5 wt.-% of a polymeric film forming agent mixture consisting of polyamide 3 and acrylate/octylacrylamide copolymer; and

e) 1 to 3.5 wt.-% of a cosmetic additive mixture consisting of perfume, tocopherol tocopheryl acetate and optionally glycerin, based on the total weight of the formulation;

wherein the water content present in the formulation is less than 1 wt.-%, based on the total weight of the formulation, and the sum of the weight of the components is 100 %.

In an even more preferred embodiment of the invention, the cosmetic formulation consists of the mixtures of compounds as defined above and the cosmetic formulation is water free.

In particular, it is preferred that the electrostatically sprayable cosmetic formulation consists of

a) 40 to 55 wt.-% ethanol;

b) 18 to 27 wt.-% a emollients mixture, which consists of 10 to 12 wt.-% phenoxyethyl caprylate, 0.5 to 2.5 wt.-% PPG-3 myristyl ether, 4.0 to 6.5 wt.% dibutyl adipate and 3.5 to 6 wt.-% dicapryl carbonate;

c) 16 to 36 wt.-% of UV-A/UV-B a filter mixture, which consists of 3 to 5.5 wt.-% butyl methoxydibenzoylmethane, 7.5 to 11 wt.-% octocrylene, 1.0 to 4.5 wt.-% diethylhexyl butamido triazone, 4.5 to 6.0 wt.-% ethylhexyl salicylate, 0 to 3.0 wt.-% bisethylhexyloxyphenol methoxyphenyl triazine, 0 to 1.5 to 2.5 wt.-% diethylamino hydroxybenzoyl hexyl benzoate and optionally 0 to 3.5 wt.-% homosalate;

d) 2 to 5 wt.-% of a polymeric film forming agent mixture, which consists of 1.5 to 3.5 wt.-% polyamide 3, and 0.5 to 1.5 wt.-% acrylate/octylacrylamide copolymer; and

e) 0.5 to 4.0 wt.-% of a cosmetic additive mixture, which consists of 0 to 2.5 wt.% glycerin, 0.25 to 0.60 wt.-% perfume, 0.1 to 0.3 wt.-% tocopherol and 0.25 to 0.60 wt.-% tocopheryl acetate, based on the total weight of the formulation;

wherein the sum of the weight of the components is 100 % and the cosmetic formulation is water free.

The examples that follow are intended for illustrating the invention in more detail.

## Examples

### 1. Measurement methods

#### 1.1 Specific electrical resistance

The specific electrical resistance was determined by the means of an Electrospray Paint Test Meter of DCA Electronic Ltd. The measuring current was between 85 $\mu$A and 100 $\mu$A or higher. The measuring range was 10 kOhm up to 5 MOhm. The measured electrical resistance was approximately conversed to the specific electrical resistance according to ASTDM D5682-08.

#### 1.2 Surface tension

The surface tensions of the formulations as described herein were measured by two different measurement methods, the stamp method and the bubble pressure method. Due to the low viscosity of the cosmetic formulations according to the invention, the stamp method was used to determine the surface tension of these cosmetic formulations.

#### 1.2.1 Stamp method

The metal stamp can be used to determine the surface tension of liquids and highly viscous media (e.g. polymer melt or adhesives). The liquid is applied to the stamp, which forms an entire surface and a drop on the stamp. The stamp material has no influence on the measurement result.

The surface tension of the liquid can be calculated from the drop contour and the density of the liquid using the SCA 20 software (www.dataphysics-instruments.com, DATAPhysics OCA20) and the Young-Laplace equation.

In addition to the surface tension, the contact angle and the volume of the drop are also measured. The determined surface tension is independent of the contact angle. The same surface tension values are measured both when the droplet is enlarged and when it is reduced by suction.

*1.2.2 Bubble pressure method*

With the bubble pressure method the dynamic surface tension of the formulations in liquids can be determined. This method is carried out by using the tensiometer SITA pro line t15 (www.sita-messtechnik.de), wherein an air stream is lead into the sample liquid through a robust capillary and the pressure development necessary for the bubble generation is measured at room temperature (automatic mode: bubble lifetime from 0.02 to 20 s. It is preferred to indicate the surface tension s [mN/m] at a bubble lifetime of 20 s.

1.3 Relative permittivity

The relative permittivity was determined by the means of a High Frequency Liquid Dielectric Constant Meter (Time Domain Reflection) "ALPHA TDR-5000" of Zadow Electronics (ww.zadow-electronics.de) having two parallel wire sensors that have a length of 5 cm. The measurement method was carried out such that the TDR-500 periodically sends voltage pulses with times (250 ps) onto the sensor cable to determine the dielectric constant (K) of the formulations:

$$K = 15^2 \times (\frac{t}{r})^2$$

t = running time in ns; the running time was 5 ns
r = length of the wire sensor (5 cm)

The relative permittivity of the formulation was calculated on the basis of the determined dielectric constant.

1.4 Electrical conductivity

The electrical conductivity was measured by means of Sartorious PT-20, hand-held conductivity meter, measuring cell PY-CL1 (www.sartorius.de). Measurement at room temperature.

1.5 Viscosity

The viscosity was determined by means of a Universal Dynamic spectrometer "Physica UDS 200" at shear rates of 1 to 3000 s$^{-1}$ and 22 °C.

1.6 Spraying test 1

In this spraying test, the sprayability of cosmetic formulations to provide a homogenous distribution on the skin surface was tested.
The conditions for all test setups were the same. The tested cosmetic formulations were sprayed by using an IONIQ spraying system, which is described in detail in DE 10 2017 108 610.2, DE 10 2017 108 612.9, DE 10 2017 108 613.7, DE 10 2107 108 614.5 and.DE 10 2107 108 615.3. The tested cosmetic formulations were sprayed on the forearms of 3 and 4, respectively, subjects. The spraying time was 0.75 s (scale 1.5) and the voltage of the pump for spraying was 2.6 V. After spraying the IONIQ system was cleaned with denaturized alcohol. The formulations were leak to soak for 30 minutes without further treatment or massage. Afterwards the homogenous distribution of the formulations on the skin was determined *in vivo* by laser scanning microscopy (LSM). The tested cosmetic formulations contained fluorescein and thus by illumination at 488 nm the fluorescence of the fluorescein could be made visible. A mosaic measuring 4 × 4 mm on the skin surface was recorded at each measuring point.

1.7 Spraying test 2

In this spraying test, also the IONIQ spray system was used and the conditions for all test setups were the same. The tested cosmetic formulations were sprayed on the forearm of a subject and were leak to soak without any further treatment. After 1.5 and 30 minutes the distribution of the formulations on the skin was photographed with a UV camera. Afterwards, the uniformity of the dark coloring was assessed.

1.8 Sun Protection Factor (SPF)

The SPF was determined according to the International standard ISO 24444:2010(E); all measurement conditions as indicated in this International standard were considered.

1.9 Water Resistance (Screening)

For the determination of water resistance of the formulation the SPF testing according to ISO 24444:2010 (E) was carried out twice at each subject. On one side of the back of the subject an SPF measurement (screening) according to ISO 24444:2010 (E), as described above, is carried out, whereas on the contra lateral side SPF testing is combined with a standardized watering procedure. Watering of the test area takes place in accordance with the COLIPA Guidelines of Evaluating Sun Product Water Resistance 2005.

2. Cosmetic Formulations

All used raw materials are cosmetic qualities and commercially available. The used ethanol was denaturized quality (denaturant: isopropyl and tert.-butanol; Ethanol 96%) and the used glycerin was Glycerin Ph. Eur. 99.8%. Both compounds are widely used in cosmetic products, which should be water free.

The components of each formulation were mixed together such that a homogeneous mixture of the formulation was obtained.

Three cosmetic formulations according to the invention, indicated as Formulation SPF 50, SPF 30 and SPF 15, were prepared. The compositions of the formulations are given in Table 2 below.

Furthermore, six cosmetic formulations, which are not in accordance with invention and indicated as Formulations of Comparative Examples 1-6 were prepared. The compositions of these formulations are given in Tables 3 and 4 below.

Table 2

| Material | INCI (EU) | SPF 50 [wt.-%] | SPF 30 [wt.-%] | SPF 15 [wt.-%] |
|---|---|---|---|---|
| Eusolex 9020 | BUTYL METHOXYDIBENZOYLMETHANE | 5 | 5 | 3.5 |
| Neo Heliopan 303 | OCTOCRYLENE | 10 | 10 | 8 |
| Uvasorb HEB | DIETHYLHEXYL BUTAMIDO TRIAZONE | 4 | 3 | 1.5 |
| Neo Heliopan OS | ETHYLHEXYL SALICYLATE | 5 | 5 | 5 |
| Parsol Shield | BISETHYLHEXYLOXYPHENOL METHOXYPHENYL TRIAZINE | 2 | 1.5 | |
| Uvinul A Plus | DIETHYLAMINO HYDROXYBENZOYL HEXYL BENZOATE | 2 | | |
| Eusolex HMS | HOMOSALATE | 3 | | |

(continued)

| Material | INCI (EU) | SPF 50 [wt.-%] | SPF 30 [wt.-%] | SPF 15 [wt.-%] |
|---|---|---|---|---|
| Total amount of UV filters | | 31 | 24.5 | 18 |
| Tegosoft XC | PHENOXYETHYLCAPRYLATE | 11.6 | 11.6 | 11.6 |
| Tegosoft APM | PPG-3 MYRISTYL ETHER | 2 | 1 | 1 |
| Cetiol B | DIBUTYL ADIPATE | 6 | 5 | 5 |
| Cetiol CC | DICAPRYLYL CARBONATE, TOCOPHEROL | 4.2 | 5.2 | 5.2 |
| Total amount of emollients | | 23.8 | 22.8 | 22.8 |
| OleoCraft HP-31 | POLYAMIDE-3 | 3 | 3 | 3 |
| Dermacryl 79 | ACRYLATES/OCTYLACRYLAMIDE COPOLYMER | 1 | 1 | 1 |
| Total amount of film forming agents | | 4 | 4 | 4 |
| Ethanol | ALCOHOL DENAT. | 40 | 45.5 | 52 |
| Glycerin | GLYCERIN | | 2 | 2 |
| Perfume | | 0.5 | 0.5 | 0.5 |
| DL-alpha-Tocopherol | TOCOPHEROL | 0.2 | 0.2 | 0.2 |
| DL-Alpha-Tocopherolacetate | TOCOPHERYL ACETATE | 0.5 | 0.5 | 0.5 |
| Total amount of additives | | 1.2 | 3.2 | 3.2 |
| SPF | | | 39.5 | |

Table 3

| Material | INCI (EU) | Comp. Ex. 1 | Comp. Ex. 2 | Comp. Ex. 3 |
|---|---|---|---|---|
| | | Formulation [wt.-%] | Formulation [wt.-%] | Formulation [wt.-%] |
| Eusolex 9020 | BUTYL METHOXYDIBENZOYLMETHANE | 4 | 4 | 4 |
| Neo Heliopan 303 | OCTOCRYLENE | 10 | 10 | 10 |
| Uvasorb HEB | DIETHYLHEXYL BUTAMIDO TRIAZONE | 2 | 2 | 2 |
| Neo Heliopan OS | ETHYLHEXYL SALICYLATE | 5 | 5 | 5 |
| Parsol Shield | BISETHYLHEXYLOXYPHENOL METHOXYPHENYL TRIAZINE | 1.5 | 1.5 | 1.5 |
| Total amount of UV filters | | 22.5 | 22.5 | 22.5 |
| Tegosoft XC | PHENOXYETHYL CAPRYLATE | 13.8 | 13.8 | 11.8 |
| Tegosoft APM | PPG-3 MYRISTYL ETHER | 5 | 5 | 5 |
| Cetiol B | DIBUTYL ADIPATE | 5 | 5 | 5 |
| Total amount of emollients | | 23.8 | 23.8 | 21.8 |
| OleoCraft HP-31 | POLYAMIDE-3 | 2 | - | - |

11

(continued)

| Material | INCI (EU) | Comp. Ex. 1 | Comp. Ex. 2 | Comp. Ex. 3 |
|---|---|---|---|---|
| | | Formulation [wt.-%] | Formulation [wt.-%] | Formulation [wt.-%] |
| Dermacryl 79 | ACRYLATES/OCTYLACRYL AMIDE COPOLYMER | - | 2 | - |
| Lexfilm Spray | POLYESTER-10, PROPYLENE GLYCOL DIBENZOATE | - | - | 4 |
| Total amount of film forming agents | | 2 | 2 | 4 |
| Ethanol | ALCOHOL DENAT. | 51 | 51 | 51 |
| Vitamin E Acetat Care | TOCOPHERYL ACETATE | 0.5 | 0.5 | 0.5 |
| Fluorescein sodium salt | | 0.2 | 0.2 | 0.2 |

Table 4

| Material | INCI (EU) | Comp. Ex. 4 | Comp. Ex. 5 | Comp. Ex.6 |
|---|---|---|---|---|
| | | Formulation [wt.-%] | Formulation [wt.-%] | Formulation [wt.-%] |
| Eusolex 9020 | BUTYL METHOXYDIBENZOYLMETHANE | 5 | 5 | 5 |
| Neo Heliopan 303 | OCTOCRYLENE | 10 | 10 | 10 |
| Uvasorb HEB | DIETHYLHEXYL BUTAMIDO TRIAZONE | 2 | 2 | 2 |
| Neo Heliopan OS | ETHYLHEXYL SALICYLATE | 5 | 5 | 5 |
| Parsol Shield | BISETHYLHEXYLOXYPHENOL METHOXYPHENYL TRIAZINE | 1 | 1 | 1 |
| Total amount of UV filters | | 23 | 23 | 23 |
| Tegosoft XC | PHENOXYETHYLCAPRYLATE | 10 | 10 | 10 |
| Tegosoft APM | PPG-3 MYRISTYL ETHER | 1 | 1 | 1 |
| Cetiol B | DIBUTYL ADIPATE | 5 | 5 | 5 |
| Cetiol CC | DICAPRYLYL CARBONATE, TOCOPHEROL | 5 | 5 | 5 |
| Total amount of emollients | | 21 | 21 | 21 |
| OleoCraft HP-31 | POLYAMIDE-3 | 6 | 3 | 3 |
| Baycusan C 2000 | POLYURETHANE-64 | | 2 | |

(continued)

| Material | INCI (EU) | Comp. Ex. 4 | Comp. Ex. 5 | Comp. Ex.6 |
|---|---|---|---|---|
| | | Formulation [wt.-%] | Formulation [wt.-%] | Formulation [wt.-%] |
| Dermacyl 79 | ACRYLATES/OCTYLACRYLAMIDE COPOLYMER | - | - | - |
| Wet Film | TRIMETHYLPENTANEDIOL/ADIPIC ACID/ GLYCERIN CROSSPOLYMER | | | 2 |
| Total amount of film forming agents | | 6 | 5 | 5 |
| Ethanol | ALCOHOL DENAT. | 47 | 48 | 48 |
| Perfume | | 0.3 | | |
| SPF | | 24 | 27.9 | 26.3 |

### 3. Results

3.1 Spraying test 1

Spraying test 1 was carried out with the cosmetic Formulations of Comparative Examples 1-3. All three formulations only include one type of film forming agent instead of a film forming agent mixture, as required in the invention.

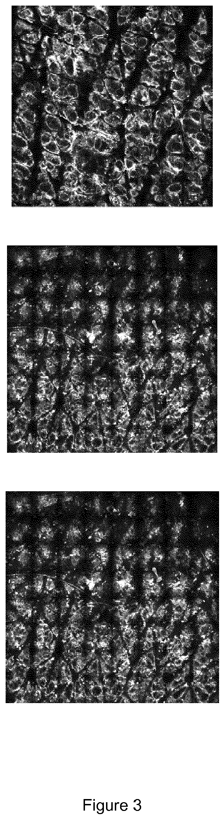

The formulation of Comparative Example 1 includes polyamide 3, i.e. a polyalkyleneoxy terminated polyamide film forming agent. The Formulation of Comparative Example 2 includes Dermacryl 79, i.e. an acrylate film forming agent, and the Formulation of Comparative Example 2 includes polyester10/Propylene glycol dibenzoate as film forming agent. In Figures 3-5 the test results are depicted.

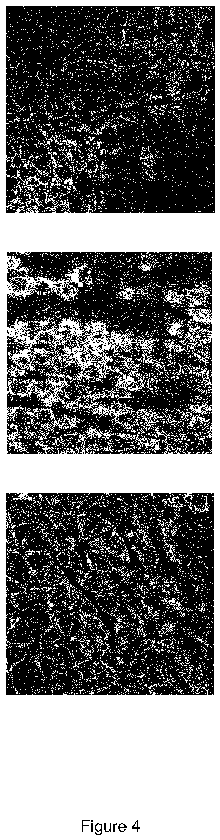

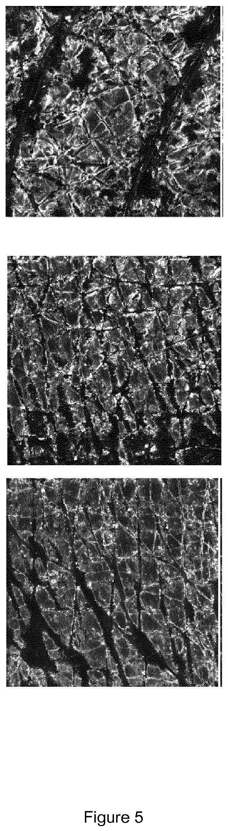

All three formulations were electrostatically sprayable and, according to the first impression, showed a homogenous distribution on the surface of the skin. A detail examination of the results showed that the Formulation of Comparative Example 1, which includes polyamide 3 as film forming agent, was most homogeneously applied (see Figure 3). However, the Formulations of Comparative Example 2 and 3 showed inhomogeneous areas (see Figure 4 and 5).

Hence, the use of a polyalkyleneoxy polyamide as film forming agent provided the best spraying result.

3.2 Spraying test 2

Figure 7 shows the spraying results of the Formulation SPF 30 according to the invention (right side) and of the Formulation of Comparative Example 4, which has a nearly similar composition of compounds as the Formulation of SPF 30 but which only includes polyamide 3 as film forming agent instead of a film forming agent mixture. In all tests the same application quantity was used.

The UVA images clearly show that the Formulation SPF 30 provided a more homogeneous distribution on the skin than the Formulation of Comparative Example 4.

Formulations SPF 15 and SPF 50 of the invention showed the same good sprayability as the Formulation of SPF 30.

3.3 Sun protection factor

The SPFs of the Formulations of Comparative Examples 4-6 and of the Formulation according to the invention were determined. The Formulation of the comparative examples and of Formulation SPF 30 included the same UV filter mixture.

As the Formulation of Comparative Example 1, the Formulation of Comparative Example 4 includes polyamide 3 as film forming agent. Although, the amount of polyamide 3 was very high in this formulation (6 wt.-% based on the total weight of the composition), only a SPF of 24 was obtained.

In contrast to the Formulation of Comparative Example 4, the Formulations of Comparative Examples 5 and 6 included film forming agent mixtures. In Comparative Example 5 a mixture of polyamide 3 and polyurethane-64 was used and in Comparative Example 6 a mixture of polyamide 3 and trimethylpentanediol/adipic acid/glycerin crosspolymer was used. However, although these formulations included film forming agent mixtures, the SPFs of the formulations were not significantly increased in comparison to the SPF of the Formulation of Comparative Example 4 (SPF of Comparative Example 5 was 27.9 and SPF of Comparative Example 6 was 26.3).

Only by using a film forming agent mixture according to the invention, i.e. a mixture which includes a polyalkyleneoxy polyamide (polyamide 3) and an acrylate (Dermacyl 79), the SPF of the cosmetic formulation was significantly increased (see Formulation SPF 30 which has an SPF of 39.5). The same applies to Formulation SPF 15 and 50.

3.4 Water Resistance

The Formulations SPF 15, 30 and 50 of the invention show a sufficient water resistance. In particular, the Formulation SPF 15 has a water resistance of 75% and the Formulation SPF 30 of 66.9%.

3.5 Relative Permittivity, specific electrical resistance and surface tension

| Property | SPF 50 | SPF 30 | SPF 15 |
|---|---|---|---|
| Relative permittivity | 2.43 | 2.62 | 2.87 |
| Specific electrical resistance [$GOhm \cdot mm^2/m$] | 46.2 | 39.6 | 42.2 |
| Surface tension (stamp method) [mN/m] | 25.10 (density: 0.9187 [$g/cm^3$]) | 24.49 (density: 0.9039 [$g/cm^3$]) | 24.57 (density: 0.8844 [$g/cm^3$]) |

3.6 Conclusion

The use of a film forming agent mixture including a polyalkyleneoxy polyamide and acrylate in a cosmetic formulation as described above, provides a cosmetic formulation, which shows an improved electrostatic sprayability, and further results in an increase of the sun screen protection factor without increasing the content of UV filters present in the formulation.

**Claims**

1. Electrostatically sprayable cosmetic formulation, comprising

   a) 20 to 60 wt.-% ethanol;
   b) 10 to 55 wt.-% emollients, which are liquid at 20 °C and have a log $K_{ow}$ of 2.0 to 10;
   c) 10 to 55 wt.-% of UV-A and/or UV-B filters;
   d) 0.5 to 15 wt.-% of a polymeric film forming agent mixture comprising at least one polyalkyleneoxy terminated polyamide and at least one acrylate; and
   e) 0 to 4 wt.-% of cosmetic additives, based on the total weight of the formulation; wherein the water content present in the formulation is less than 5 wt.-%, based on the total weight of the formulation, and the sum of the weight of the components is 100 %.

2. Electrostatically sprayable cosmetic formulation according to claim 1, wherein the composition does not comprise a thickening agent.

3. Electrostatically sprayable cosmetic formulation according to claims 1 and 2, wherein the amount of the polyalkyleneoxy terminated polyamide is at least twice higher than the amount of acrylate present in the film forming agent mixture.

4. Electrostatically sprayable cosmetic formulation according to any one of the previous claims, wherein the amount of polyalkyleneoxy terminated polyamide is at least 0.5 wt.% and not higher than 10 wt.-%, based on the total weight of the formulation.

5. Electrostatically sprayable cosmetic formulation according to claim 4, wherein the amount of the acrylate is at least 0.2 wt.-% and not higher than 3 wt.-%, based on the total weight of the formulation.

6.  Electrostatically sprayable cosmetic formulation according to any one of the previous claims, wherein the polyalkyleneoxy terminated polyamide is a condensation product of dilinoleic acid, ethylenediamine and polypropylene glycol diamine end-capped with polyethylene glycol-poylpropylene glycol (PEG-PPG)-32/10 aminopropyl methyl ether.

7.  Electrostatically sprayable cosmetic formulation according to any one of the previous claims, wherein the acrylate is an acrylate/amide copolymer.

8.  Electrostatically sprayable cosmetic formulation according to any one of the previous claims, wherein the cosmetic formulation comprises at least two different emollients.

9.  Electrostatically sprayable cosmetic formulation according to any one of the previous claims, wherein the emollients are selected form the group consisting of phenoxyethyl caprylate, PPG-3 myristyl ether, dibutyl adipate, dicaprylyl carbonate, isoamyl cocoate, diethylhexyl carbonate, isopropyl myristate, isopropyl palmitate, PPG-14 butyl ether, decyl cocoate, PPG-15 stearyl ether, $C_{12}$-$C_{15}$ alkyl benzoate and combinations thereof.

10. Electrostatically sprayable cosmetic formulation according to any one of the previous claims, wherein the UV-A and/or UV-B filters having log $K_{ow}$ of 2.0 to 7.0.

11. Electrostatic sprayable cosmetic formulation according to any one of the previous claims, wherein the UV-A and/or UV-B filters are selected from the group consisting of butyl methoxydibenzoylmethane, octocrylene, diethylhexyl butamido triazone, ethylhexyl salicylate, bis-ethylhexyloxyphenol methoxyphenyl triazine, diethylamino hydroxybenzoyl hexyl benzoate, homosalate and combinations thereof.

12. Electrostatically sprayable cosmetic formulation according to any one of the previous claims, wherein the cosmetic additives are selected from the group consisting of moisture agents, perfumes, vitamin, pro-vitamins and combinations thereof.

13. Electrostatically sprayable cosmetic formulation according to any one of the previous claims, wherein the cosmetic formulation is a sun screen formulation.

14. Electrostatically sprayable cosmetic formulation according to any one of the previous claims, wherein the cosmetic formulation comprises

    a) 35 to 55 wt.-% ethanol;
    b) 18 to 27 wt.-% a emollients mixture, which consists of phenoxyethyl caprylate, PPG-3 myristyl ether, dibutyl adipate and dicaprylyl carbonate;
    c) 16 to 40 wt.-% of a UV-A/UV-B filter mixture, which consists of butyl methoxydibenzoylmethane, octocrylene, diethylhexyl butamido triazone, ethylhexyl salicylate, optionally bis-ethylhexyloxyphenol methoxyphenyl triazine, optionally diethylamino hydroxybenzoyl hexyl benzoate and optionally homosalate;
    d) 2 to 5 wt.-% of a polymeric film forming agent mixture, which consists of polyamide 3 and acrylate/octylacrylamide copolymer; and
    e) 1 to 3.5 wt.-% of a cosmetic additive mixture, which consists of perfume, tocopherol, tocopheryl acetate and optionally glycerin, based on the total weight of the formulation,

    wherein the water content present in the formulation is less than 1 wt.-%, based on the total weight of the formulation, and the sum of the weight of the components is 100 %.

15. The use of a film forming agent mixture comprising at least one polyalkyleneoxy terminated polyamide and at least one acrylate, in a cosmetic formulation comprising

    a) 20 to 60 wt.-% ethanol;
    b) 10 to 55 wt.-% emollients, which are liquid at 20 °C and have a log $K_{ow}$ of 2.0 to 10
    c) 10 to 55 wt.-% of UV-A and/or UV-B filters; and
    e) 0 to 4 wt.-% of cosmetic additives, based on the total weight of the formulation, wherein the water content present in the formulation is less than 5 wt.-%, based on the total weight of the formulation, and the sum of the weight of the components is 100 %,

for electrostatic spraying of the cosmetic formulation.

**Patentansprüche**

1. Elektrostatisch sprühbare kosmetische Formulierung, umfassend

a) 20 bis 60 Gew.-% Ethanol;
b) 10 bis 55 Gew.-% Erweichungsmittel, die bei 20 °C flüssig sind und einen log $K_{ow}$ von 2,0 bis 10 haben;
c) 10 bis 55 Gew.-% UV-A- und/oder UV-B-Filter;
d) 0,5 bis 15 Gew.-% eines Polymerfilmbildnergemisches, umfassend mindestens ein Polyalkylenoxy-terminiertes Polyamid und mindestens ein Acrylat; und
e) 0 bis 4 Gew.-% kosmetischer Additive, basierend auf dem Gesamtgewicht der Formulierung;

wobei der Wassergehalt in der Formulierung weniger als 5 Gew.-%, basierend auf dem Gesamtgewicht der Formulierung, beträgt und die Summe des Gewichts der Komponenten 100 % beträgt.

2. Elektrostatisch sprühbare kosmetische Formulierung nach Anspruch 1, wobei die Zusammensetzung kein Verdickungsmittel umfasst.

3. Elektrostatisch sprühbare kosmetische Formulierung nach den Ansprüchen 1 und 2, wobei die Menge des Polyalkylenoxy-terminierten Polyamids mindestens zweimal höher als die Menge an Acrylat in dem Filmbildnergemisch ist.

4. Elektrostatisch sprühbare kosmetische Formulierung nach einem der vorstehenden Ansprüche, wobei die Menge an Polyalkylenoxy-terminiertem Polyamid wenigstens 0,5 Gew.-% und nicht mehr als 10 Gew.-%, basierend auf dem Gesamtgewicht der Formulierung, beträgt.

5. Elektrostatisch sprühbare kosmetische Formulierung nach Anspruch 4, wobei die Menge des Acrylats wenigstens 0,2 Gew.-% und nicht mehr als 3 Gew.-%, basierend auf dem Gesamtgewicht der Formulierung, beträgt.

6. Elektrostatisch sprühbare kosmetische Formulierung nach einem der vorstehenden Ansprüche, wobei das Polyalkylenoxy-terminierte Polyamid ein Kondensationsprodukt von Dilinolsäure, Ethylendiamin und Polypropylenglycoldiamin, endgruppengeschützt mit Polyethylenglycol-Polypropylenglycol- (PEG-PPG)-32/10-Aminopropylmethylether, ist.

7. Elektrostatisch sprühbare kosmetische Formulierung nach einem der vorstehenden Ansprüche, wobei das Acrylat ein Acrylat/Amid-Copolymer ist.

8. Elektrostatisch sprühbare kosmetische Formulierung nach einem der vorstehenden Ansprüche, wobei die kosmetische Formulierung mindestens zwei verschiedene Erweichungsmittel umfasst.

9. Elektrostatisch sprühbare kosmetische Formulierung nach einem der vorstehenden Ansprüche, wobei die Erweichungsmittel ausgewählt sind aus der Gruppe, bestehend aus Phenoxyethylcaprylat, PPG-3-Myristylether, Dibutyladipat, Dicaprylylcarbonat, Isoamylcocoat, Diethylhexylcarbonat, Isopropylmyristat, Isopropylpalmitat, PPG-14-Butylether, Decylcocoat, PPG-15-Stearylether, $C_{12}$-$C_{15}$-Alkylbenzoat und Kombinationen davon.

10. Elektrostatisch sprühbare kosmetische Formulierung nach einem der vorstehenden Ansprüche, wobei die UV-A- und/oder UV-B-Filter einen log $Ko_w$ von 2,0 bis 7,0 haben.

11. Elektrostatisch sprühbare kosmetische Formulierung nach einem der vorstehenden Ansprüche, wobei die UV-A- und/oder UV-B-Filter ausgewählt sind aus der Gruppe, bestehend aus Butylmethoxydibenzoylmethan, Octocrylen, Diethylhexylbutamidotriazon, Ethylhexylsalicylat, Bisethylhexyloxyphenolmethoxyphenyltriazin, Diethylaminohydroxybenzoylhexylbenzoat, Homosalat und Kombinationen davon.

12. Elektrostatisch sprühbare kosmetische Formulierung nach einem der vorstehenden Ansprüche, wobei die kosmetischen Additive ausgewählt sind aus der Gruppe, bestehend aus Feuchthaltemitteln, Geruchsstoffen, Vitamin, Pro-Vitaminen und Kombinationen davon.

**13.** Elektrostatisch sprühbare kosmetische Formulierung nach einem der vorstehenden Ansprüche, wobei die kosmetische Formulierung eine Sonnenschutzformulierung ist.

**14.** Elektrostatisch sprühbare kosmetische Formulierung nach einem der vorstehenden Ansprüche, wobei die kosmetische Formulierung

a) 35 bis 55 Gew.-% Ethanol;
b) 18 bis 27 Gew.-% eines Erweichungsmittelgemisches, das aus Phenoxyethylcaprylat, PPG-3-Myristylether, Dibutyladipat und Dicaprylylcarbonat besteht;
c) 16 bis 40 Gew.-% eines UV-A/UV-B-Filtergemisches, das aus Butylmethoxydibenzoylmethan, Octocrylen, Diethylhexylbutamidotriazon, Ethylhexylsalicylat, gegebenenfalls Bisethylhexyloxyphenolmethoxyphenyltriazin, gegebenenfalls Diethylaminohydroxybenzoylhexylbenzoat und gegebenenfalls Homosalat besteht;
d) 2 bis 5 Gew.-% eines Polymerfilmbildnergemisches, das aus Polyamid 3 und Acrylat/Octylacrylamid-Copolymer besteht; und
e) 1 bis 3,5 Gew.-% eines kosmetischen Additivgemisches, das aus Geruchsstoff, Tocopherol, Tocopherylacetat und gegebenenfalls Glycerin besteht, basierend auf dem Gesamtgewicht der Formulierung, umfasst,

wobei der Wassergehalt in der Formulierung weniger als 1 Gew.-%, basierend auf dem Gesamtgewicht der Formulierung, beträgt und die Summe des Gewichts der Komponenten 100 % beträgt.

**15.** Verwendung eines Filmbildnergemisches, umfassend mindestens ein Polyalkylenoxy-terminiertes Polyamid und mindestens ein Acrylat, in einer kosmetischen Formulierung, umfassend

a) 20 bis 60 Gew.-% Ethanol;
b) 10 bis 55 Gew.-% Erweichungsmittel, die bei 20 °C flüssig sind und einen log $K_{ow}$ von 2,0 bis 10 haben;
c) 10 bis 55 Gew.-% UV-A- und/oder UV-B-Filter und
e) 0 bis 4 Gew.-% kosmetischer Additive, basierend auf dem Gesamtgewicht der Formulierung; wobei der Wassergehalt in der Formulierung weniger als 5 Gew.-%, basierend auf dem Gesamtgewicht der Formulierung, beträgt und die Summe des Gewichts der Komponenten 100 % beträgt,

zum elektrostatischen Sprühen der kosmetischen Formulierung.

**Revendications**

**1.** Formulation cosmétique pulvérisable électrostatiquement, comprenant

a) 20 à 60 % en poids d'éthanol ;
b) 10 à 55% en poids d'émollients, qui sont liquides à 20°C et ont un log $K_{ow}$ de 2,0 à 10 ;
c) 10 à 55 % en poids de filtres UV-A et/ou UV-B ;
d) 0,5 à 15 % en poids d'un mélange d'agents de formation de film polymérique comprenant au moins un polyamide à terminaison polyalkylèneoxy et au moins un acrylate ; et
e) 0 à 4 % en poids d'additifs cosmétiques, par rapport au poids total de la formulation ;

dans laquelle la teneur en eau présente dans la formulation est inférieure à 5 % en poids, sur la base du poids total de la formulation, et la somme des poids des composants est de 100 %.

**2.** Formulation cosmétique pulvérisable électrostatiquement selon la revendication 1, dans laquelle la composition ne comprend pas d'agent épaississant.

**3.** Formulation cosmétique pulvérisable électrostatiquement selon les revendications 1 et 2, dans laquelle la quantité de polyamide à terminaison polyalkylèneoxy est au moins deux fois supérieure à la quantité d'acrylate présente dans le mélange d'agents filmogènes.

**4.** Formulation cosmétique pulvérisable électrostatiquement selon l'une quelconque des revendications précédentes, dans laquelle la quantité de polyamide à terminaison polyalkylèneoxy est d'au moins 0,5 % en poids et ne dépasse pas 10 % en poids, par rapport au poids total de la formulation.

5. Formulation cosmétique pulvérisable électrostatiquement selon la revendication 4, dans laquelle la quantité d'acrylate est d'au moins 0,2 % en poids et n'est pas supérieure à 3 % en poids, par rapport au poids total de la formulation.

6. Formulation cosmétique pulvérisable électrostatiquement selon l'une quelconque des revendications précédentes, dans laquelle le polyamide à terminaison polyalkylèneoxy est un produit de condensation d'acide dilinoléique, d'éthylènediamine et de polypropylène glycol diamine coiffé d'un éther aminopropylméthylique polyéthylène glycol-poylpropylène glycol (PEG-PPG)-32/10.

7. Formulation cosmétique pulvérisable électrostatiquement selon l'une quelconque des revendications précédentes, dans laquelle l'acrylate est un copolymère acrylate/amide.

8. Formulation cosmétique pulvérisable électrostatiquement selon l'une quelconque des revendications précédentes, dans laquelle la formulation cosmétique comprend au moins deux émollients différents.

9. Formulation cosmétique pulvérisable électrostatiquement selon l'une quelconque des revendications précédentes, dans laquelle les émollients sont choisis dans le groupe constitué par le caprylate de phénoxyéthyle, le PPG-3 myristyl éther, l'adipate de dibutyle, le carbonate de dicaprylyle, le cocoate d'isoamyle, le carbonate de diéthylhexyle, le myristate d'isopropyle, le palmitate d'isopropyle, l'éther butylique PPG-14, le cocoate de décyle, l'éther stéarylique PPG-15, le benzoate d'alkyle en $C_{12}$-$C_{15}$ et leurs combinaisons.

10. Formulation cosmétique pulvérisable électrostatiquement selon l'une quelconque des revendications précédentes, dans laquelle les filtres UV-A et/ou UV-B ont un log $K_{ow}$ de 2,0 à 7,0.

11. Formulation cosmétique pulvérisable électrostatiquement selon l'une quelconque des revendications précédentes, dans laquelle les filtres UV-A et/ou UV-B sont choisis dans le groupe constitué par le butyl méthoxydibenzoylméthane, l'octocrylène, la diéthylhexyl butamido triazone, le salicylate d'éthylhexyle, la biséthylhexyloxyphénol méthoxyphényl triazine, le diéthylamino hydroxybenzoyl hexyl benzoate, l'homosalate et leurs combinaisons.

12. Formulation cosmétique pulvérisable électrostatiquement selon l'une quelconque des revendications précédentes, dans laquelle les additifs cosmétiques sont choisis dans le groupe constitué par les agents hydratants, les parfums, les vitamines, les pro-vitamines et leurs combinaisons.

13. Formulation cosmétique pulvérisable électrostatiquement selon l'une quelconque des revendications précédentes, dans laquelle la formulation cosmétique est une formulation d'écran solaire.

14. Formulation cosmétique pulvérisable électrostatiquement selon l'une quelconque des revendications précédentes, dans laquelle la formulation cosmétique comprend

   a) 35 à 55 % en poids d'éthanol ;
   b) 18 à 27 % en poids d'un mélange d'émollients, qui consiste en caprylate de phénoxyéthyle, éther myristylique PPG-3, adipate de dibutyle et carbonate de dicaprylyle ;
   c) 16 à 40 % en poids d'un mélange de filtres UV-A/UV-B, constitué de butylméthoxydibenzoylméthane, d'octocrylène, de diéthylhexylbutamidotriazone, de salicylate d'éthylhexyle, éventuellement de biséthylhexyloxyphénol-méthoxyphényl-triazine, éventuellement de diéthylaminohydroxybenzoyl-hexyl-benzoate et éventuellement d'homosalate ;
   d) 2 à 5 % en poids d'un mélange d'agents de formation de film polymérique, qui consiste en un polyamide 3 et un copolymère d'acrylate/octylacrylamide ; et
   e) 1 à 3,5 % en poids d'un mélange d'additifs cosmétiques, qui consiste en parfum, tocophérol, acétate de tocophérol et éventuellement glycérine, par rapport au poids total de la formulation,

   dans laquelle la teneur en eau présente dans la formulation est inférieure à 1 % en poids, sur la base du poids total de la formulation, et la somme du poids des composants est de 100 %.

15. Utilisation d'un mélange d'agents formant de film comprenant au moins un polyamide à terminaison polyalkylèneoxy et au moins un acrylate, dans une formulation cosmétique comprenant

   a) 20 à 60 % en poids d'éthanol ;
   b) 10 à 55 % en poids d'émollients, qui sont liquides à 20 °C et ont un log $K_{ow}$ de 2,0 à 10

c) 10 à 55 % en poids de filtres UV-A et/ou UV-B ; et

e) 0 à 4 % en poids d'additifs cosmétiques, par rapport au poids total de la formulation, dans laquelle la teneur en eau présente dans la formulation est inférieure à 5 % en poids, par rapport au poids total de la formulation, et la somme des poids des composants est de 100 %,

pour la pulvérisation électrostatique de la formulation cosmétique.

Figure 1

Figure 2

Figure 3

Figure 4

Figure 5

Detail A

Figure 6

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 202010006005 U1
- WO 9411119 A
- WO 2001012139 A
- WO 2018194086 A
- US 20100116897 A
- DE 102017108610
- DE 102017108612
- DE 102017108613
- DE 102107108614
- DE 102107108615
- US 20140212363 A
- WO 0112139 A

### Non-patent literature cited in the description

- SYLVASOL Polymers - Ethanol Sunscreen sprays. IP.COM Journal. IP.COM Inc, 21 February 2012
- Octanol-Water Partition Coefficients: Fundamentals and Physical Chemistry. **J. SANGSTER.** Wiley Series in solution chemistry. John Wiley & Sons, 1997, vol. 2
- *CHEMICAL ABSTRACTS,* 288254-16-0